Europäisches Patentamt

European Patent Office

Office européen des brevets

(11) Numéro de publication: **0 315 557**
**A1**

## DEMANDE DE BREVET EUROPEEN

(21) Numéro de dépôt: **88460025.5**

(22) Date de dépôt: **25.10.88**

(51) Int. Cl.⁴: **A 61 M 1/00**

(30) Priorité: **27.10.87 FR 8715028**

(43) Date de publication de la demande:
**10.05.89 Bulletin 89/19**

(84) Etats contractants désignés:
**BE CH DE ES GB IT LI**

(71) Demandeur: **Le Cam, Daniel**
**Kerouezec**
**F-29213 Plougastel-Daoulas (FR)**

(72) Inventeur: **Le Cam, Daniel**
**Kerouezec**
**F-29213 Plougastel-Daoulas (FR)**

(74) Mandataire: **Martin, Jean-Jacques et al**
**Cabinet REGIMBEAU 11, rue Franz Heller**
**F-35700 Rennes (FR)**

(54) Dispositif de liposuccion.

(57) Dispositif de liposuccion.
Ce dispositif comprend une canule (3) destinée à être raccordée à une pompe (7) qui est adaptée pour aspirer la graisse à travers des ouvertures (30) prévues à l'extrémité de la canule lorsque celle-ci est introduite sous le peau, dans le tissu graisseux ; conformément à l'invention, il est prévu des moyens (9, 8) d'entraînement en rotation de la canule (3) autour de son propre axe (X-X').
La mise en rotation de la canule facilite les déplacements de la canule au sein du tissu graisseux, permettant au praticien de travailler en douceur, sans efforts physiques excessifs.
Matériel de chirurgie esthétique.

FIG.1

EP 0 315 557 A1

**Description**

## DISPOSITIF DE LIPOSUCCION

La présente invention concerne un dispositif de liposuccion.

La luposuccion est une technique utilisée en chirurgie esthétique depuis une dizaine d'années, qui consiste à éliminer par aspiration des accumulations de graisse pouvant se trouver en excès en certaines régions du corps humain, notamment au niveau du ventre, des fesses, des hanches et du menton.

Les dispositifs de liposuccion qui ont été développés jusqu'ici sont relativement rudimentaires. Ils comprennent une canule de forme générale tubulaire dont une extrémité présente au moins une ouverture qui traverse la paroi de la canule, et dont l'autre extrémité est raccordée par un tuyau à une pompe, via un flacon récepteur. Après avoir pratiqué une petite incision dans la région à traiter, le praticien introduit la canule sous la peau, au sein du tissu graisseux et, par un mouvement en éventail de la canule, effectue un balayage de toute la zone adipeuse accessible ; dans le même temps, la pompe réalise une succion des cellules graisseuses et celles-ci sont aspirées à travers l'ouverture de la canule pour être acheminées dans le flacon récepteur.

Si cette technique donne généralement satisfaction, en raison de sa simplicité de mise en oeuvre et de son efficacité, elle présente toutefois l'inconvénient de requérir, de la part du praticien, des efforts physiques relativement importants et parfois brutaux durant le balayage évoqué plus haut. En outre, l'élimination de la graisse ne se fait pas toujours de manière parfaitement homogène, ce qui provoque l'apparition à la surface de la région traitée d'un aspect de "vagues" très peu esthétique.

La présente invention a pour objectif de résoudre ces difficultés en proposant un dispositif de liposuccion perfectionné qui facilite la tâche du praticien en lui permettant de travailler en douceur et de façon parfaitement régulière et homogène.

Ces résultats sont atteints, conformément à l'invention, grâce au fait que la canule a une forme cylindrique et que le dispositif comporte des moyens d'entraînement en rotation de cette canule autour de son propre axe. Grâce à cet arrangement, la pénétration de la canule dans le tissu graisseux et son déplacement au sein de celui-ci sont facilités par la rotation de la canule et se font beaucoup plus aisément qu'avec les dispositifs traditionnels ; d'autre part, l'aspiration des cellules se fait de manière homogène, en toutes directions, autour de la canule.

Dans un mode de réalisation préférentiel de l'invention, la canule est montée de manière amovible à une extrémité d'une pièce tournante qui possède une forme générale tubulaire et qui est montée et guidée en rotation dans un support creux ; dans ce cas, la canule est disposée dans le prolongement de la pièce tournante tubulaire, de sorte que leurs espaces intérieurs communiquent directement l'un avec l'autre, permettant le passage des cellules graisseuses. Selon ce mode de réalisation préférentiel, le support creux présente une cavité interne destinée à être raccordée à la pompe, cette cavité constituant une chambre d'aspiration, tandis que la paroi de la pièce tournante tubulaire présente des ouvertures qui font communiquer son espace intérieur avec cette chambre d'aspiration ; la pièce tournante a par conséquent la forme d'une cage qui laisse passer les cellules graisseuses au cours de sa rotation.

Le support creux est muni d'une tubulure permettant le branchement d'un tuyau de raccordement pour faire communiquer la chambre d'aspiration avec la pompe ; ce support creux a avantageusement une forme extérieure approximativement cylindrique qui permet une prise en main commode du dispositif ; il joue donc le rôle d'un manche pour la préhension et le maniement du dispositif.

L'entraînement de la canule se fait au moyen d'un moteur, par l'intermédiaire d'un arbre d'entraînement, par exemple un arbre flexible, qui est fixé à l'extrémité de la pièce tournante qui est opposée à l'extrémité recevant la canule. Le moteur d'entraînement est avantageusement un moteur possédant deux sens possibles de rotation, dont la commande est réalisée au moyen de pédales, par l'intermédiaire d'un variateur de vitesse. Ainsi, le praticien peut commander à l'aide de son pied le sens et la vitesse de rotation de la canule, au cours de son intervention, tout en disposant de ses deux mains pour travailler.

Le dispositif est équipé de préférence d'un jeu de canules de longueur et de diamètre différents, ces canules possédant un embout identique qui permet de les monter sélectivement à l'extrémité de la pièce tournante. Grâce à cet arrangement, il est possible d'adapter simplement les dimensions de la canule au genre de l'intervention à laquelle on a affaire.

D'autres caractéristiques et avantages de l'invention apparaîtront de la description et des dessins annexés qui en présentent un mode de réalisation préférentiel.

Sur les dessins :

- la figure 1 représente le dispositif coupé par un plan de symétrie longitudinal ;
- la figure 2 est une vue de dessus de la canule équipant le dispositif de la figure 1 ;
- la figure 2A est une vue de gauche de cette canule ;
- les figures 3 et 4 sont des vues destinées à illustrer une opération de liposuccion qui est réalisée au moyen du dispositif de la figure 1, dans la région extérieure de la cuisse.

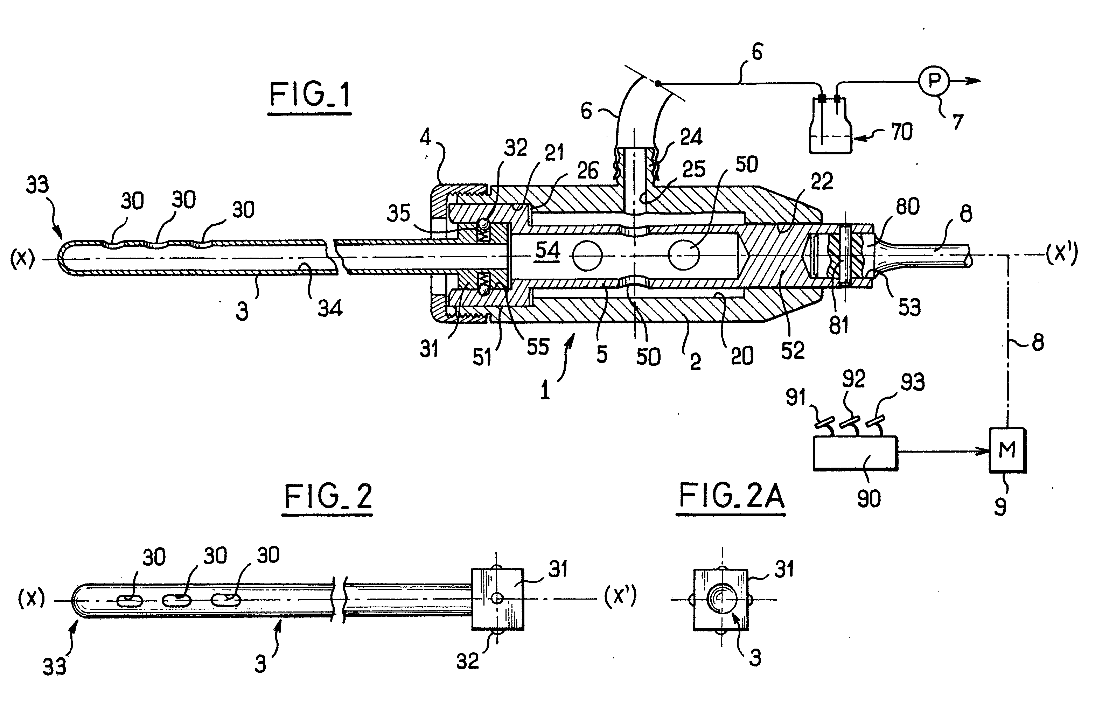

Le dispositif représenté sur la figure 1, désigné par la référence 1, comprend un corps ou support tubulaire creux, de forme générale approximativement cylindrique, désigné par la référence 2. Il présente trois alésages internes, à diamètres décroissants 21, 20 et 22 ; les alésages d'extrémité 21, 22 forment des paliers qui assurent la retenue et le guidage en rotation d'une pièce rotative 5 montée à l'intérieur du support 2 ; l'alésage central 20 constitue une chambre d'aspiration, adaptée pour être mise en communication avec une pompe 7 ; à cet effet, il est prévu sur le dessus du support 2 une tubulure 24 qui communique avec la cavité 20 par un alésage 25, cette tubulure pouvant recevoir un tuyau flexible 6. Le tuyau 6 est raccordé à la pompe 7 par l'intermédiaire d'un flacon récepteur 70.

La pièce tournante 5 est une pièce généralement tubulaire, dont la partie centrale, qui se trouve dans la cavité 20, présente des ouvertures 50 qui en traversent la paroi ; ces ouvertures, qui sont par exemple circulaires, font communiquer l'espace intérieur 54 de la pièce 5 avec l'espace de la cavité 20. Les deux extrémités 51, 52 de la pièce 5, de forme cylindrique, ont des dimensions déterminées pour assurer leur guidage en rotation dans les alésages 21, 22 respectivement du corps 2 ; du côté de la partie d'extrémité 51, est prévue une ouverture 55, de forme carrée, qui est adaptée pour recevoir par encliquetage élastique un embout d'extrémité 31 prévu sur la canule 3 ; cette canule est un tube cylindrique dont l'extrémité 33 opposée à celle qui forme l'embout 31, est fermée et arrondie (hémisphérique) ; cette même extrémité 33 présente plusieurs ouvertures 30 qui en traversent la paroi.

L'embout à contour carré 31 est muni d'une série de petites billes 32 sollicitées par des ressorts 35, et aptes à s'encliqueter élastiquement dans des petites cuvettes prévues à cet effet dans le logement 55 de la pièce 5.

L'autre extrémité 52 de la pièce 5 présente une cavité 53 adaptée pour être fixée à l'extrémité d'un arbre d'entraînement 8 ; ce dernier est par exemple un arbre flexible en matière synthétique, dont la tête 80 est emboîtée dans le logement 53, la fixation étant assurée par une goupille 81.

L'arbre 8 est entraîné par un moteur 9, et celui-ci est commandé à distance par un variateur de vitesse 90, lui-même piloté par des pédales 91, 92, 93.

Le moteur 9 et le variateur 90 sont des dispositifs connus, qui ne font pas à proprement parler partie de l'invention, et c'est pourquoi ils ont été représentés seulement de manière schématique à la figure 1.

Le moteur 9 est par exemple un moteur électrique ou pneumatique, de préférence à deux sens de rotation ; il est équipé de préférence d'un dispositif de sécurité apte à réaliser l'arrêt automatique du moteur en cas de surcharge.

Le dispositif 90 permet, à l'aide des pédales de commande, de réaliser soit l'arrêt du moteur, soit sa mise en rotation dans un sens ou dans l'autre, avec une vitesse variable, proportionnelle à la pression effectuée sur la pédale concernée.

La vitesse de rotation requise pour le moteur 9 est relativement faible, de l'ordre de 280 tr/mn en charge et de 320 tr/mn à vide.

A l'extrémité du support 2 situé du côté de la canule 3, est vissé un couvercle 4 qui empêche la pièce 5 de ressortir par ce côté ; de l'autre côté, c'est un épaulement 26 prévu dans le corps 2 qui assure la retenue en translation de la pièce 5.

Par ailleurs, il est prévu éventuellement des garnitures d'étanchéité, telles que des bagues (non représentées), qui tout en autorisant la rotation de la pièce 5 à l'intérieur du corps 2, assurent l'étanchéité de la chambre de dépression 20.

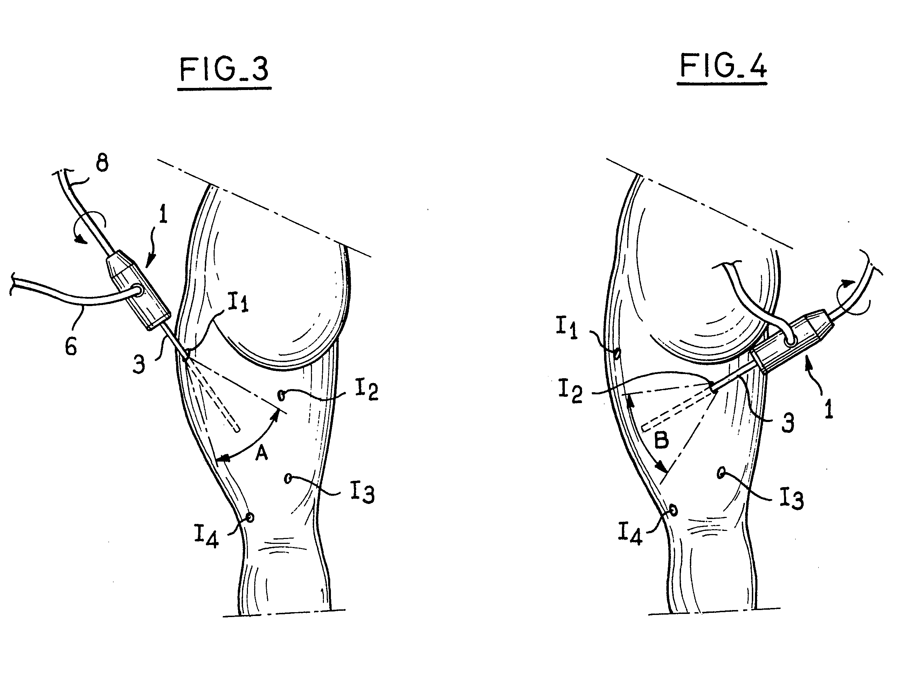

Nous allons maintenant expliquer, en nous référant notamment aux figures 3 et 4, comment le dispositif qui vient d'être décrit est utilisé pour une opération de chirurgie esthétique qui consiste à enlever la graisse en excès localisée dans la région externe de la cuisse ; un tel excès, assez fréquent chez la femme, est usuellement appelé "culotte de cheval".

Il est prévu de préférence, en association avec le dispositif qui vient d'être décrit, un jeu de canules qui possèdent des longueurs et des diamètres différents (adaptés aux types d'intervention concernés) mais sont pourvues d'un embout 31 identique, en quelque sorte "standardisé". Ainsi à titre indicatif, le diamètre de la canule sera de l'ordre de 2 mm pour un traitement au niveau des joues et du visage, de 4 mm pour un traitement sous les bras, de l'ordre de 6 à 8 mm pour un traitement au niveau du ventre, des hanches, des fesses ou des cuisses. De même, la longueur de la canule pourra varier dans une fourchette comprise entre 10 et 30 cm ; cette longueur sera par exemple de 25 cm pour une intervention dans la cuisse.

Le praticien commence par sélectionner la canule qu'il souhaite utiliser, et met en place celle-ci par encliquetage élastique dans le logement 55 de la pièce 5 ; dans cette position, l'axe X-X' de la canule correspond avec celui de la pièce rotative 5. Ensuite, il met en marche la pompe 7, de telle manière que celle-ci développe une pression d'aspiration (pression négative) de l'ordre de 600 à 1 000 mbar.

Il pratique à travers la peau, à la périphérie de la zone à traiter, une série de petites incisions à l'aide d'un bistouri, ces incisions étant destinées à permettre l'introduction de la canule dans le tissu graisseux subdermique.

Dans l'exemple représenté, il est pratiqué quatre incisions $I_1$, $I_2$, $I_3$, $I_4$ qui correspondent aux quatre sommets d'un quadrilatère à l'intérieur duquel s'inscrit la zone adipeuse à traiter.

Le praticien saisit le dispositif par le corps 2, celui-ci jouant le rôle d'un manche pour la canule 3 ; la tubulure 24 et le tuyau 6 sont de préférence dirigés vers le haut, des moyens de suspension appropriés étant prévus pour maintenir le tuyau 6 au-dessus de la table d'opération, de manière à ce qu'il ne contrarie pas le travail du praticien. Ce dernier introduit la canule 3 à travers l'une $I_1$ des incisions tout en commandant au pied, à l'aide de l'une des pédales 91, 92, 93 la rotation du moteur (9) dans un certain sens ; cette rotation est transmise à la canule 3 par l'intermédiaire de l'arbre flexible 8 et de la pièce tournante 5 ; la vitesse de rotation est de l'ordre de 300 tours par minute.

De manière similaire à ce qui avantageusement prévu pour le tuyau 6, le moteur 9 est placé de préférence au-dessus de la table d'opération, de telle sorte que l'arbre flexible 8 ne gêne pas le travail du praticien ; du reste, ce moteur peut avantageusement être porté par un joint universel, tel qu'une rotule, de manière à suivre dans une certaine mesure les mouvements donnés au dispositif par le praticien au cours de son intervention.

Lorsque la canule a été introduite sous la peau, au sein du tissu graisseux, le praticien déplace le dispositif de manière à faire effectuer à la canule 3 un mouvement longitudinal de va-et-vient et un mouvement transversal de balayage angulaire (double flèche A, figure 3). Les cellules graisseuses qui se trouvent à proximité des ouvertures 30 sont aspirées par celles-ci, pénètrent dans l'espace intérieur 34 de la canule puis dans l'espace interne 54 de la pièce tournante 5, traversent les ouvertures 50 de cette dernière pour pénétrer dans la cavité fixe 20, puis sont acheminées à travers l'alésage 25 dans le tuyau 6 jusqu'au flacon récepteur 70 où elles sont déposées.

Le mouvement de rotation de la canule facilite considérablement sa pénétration dans le tissu graisseux, et le travail du praticien est tout à fait facile ; les mouvements se font sans à-coups, ce qui réduit les risques de perforations et accroît l'efficacité ; de préférence, durant l'intervention, le praticien guide à l'aide de son autre main la canule sous la peau de manière à former un pli régulier et suffisamment large pour éviter une abrasion du tissu cellulaire sous-cutané ; ceci est relativement aisé car les mouvements de liposuccion sont lents et doux et favorisent la progression de la canule.

Lorsque la zone a été correctement balayée, le praticien retire la canule et l'introduit dans le tissu graisseux par une autre incision I₂, par laquelle il va effectuer un nouveau balayage en éventail du tissu graisseux, croisé avec le précédent (double flèche B, figure 4).

Il opère de la même manière pour les autres incisions I₃ et I₄.

Lorsque l'intervention est terminée, chaque incision est refermée par un point de suture, et il est posé un bandage autour de la région traitée, bandage qui sera conservé pendant quelques jours par le (ou la) patient(e).

Le fait que le moteur 9 puisse tourner dans les deux sens est particulièrement intéressant car il permet au praticien de faire ressortir facilement la canule d'une zone où elle aurait tendance à se bloquer, cette rotation en sens inverse s'apparentant à un dévissage qui facilite le retrait de la canule.

Par rapport à un dispositif à canule fixe, le dispositif selon l'invention est d'un maniement très aisé pour le praticien, et réalise un traitement particulièrement efficace et de bonne qualité, sans apparition de l'aspect de "vagues" évoqué plus haut.

On notera que le dispositif est facilement démontable, ce qui facilite son nettoyage et la stérilisation des différents éléments qui le composent après chaque opération. C'est ainsi qu'il est facile de retirer la pièce rotative 5 du corps 2 par simple translation axiale après dévissage du couvercle 4.

Il va de soi que diverses variantes d'exécution peuvent être envisagées. Ainsi par exemple, au lieu de prévoir un entraînement de la canule par un moteur indépendant commandé à distance, il serait possible de monter à l'extrémité du corps 2 un petit moteur portatif en prise directe avec la pièce 5.

Pour améliorer le guidage en rotation de la partie mobile 5 dans le corps 2, il serait possible de prévoir des paliers anti-friction ou des roulements à billes. Enfin, divers modes de montage, à fixation rapide, peuvent naturellement être envisagés qui permettent de monter de manière amovible la canule 3 dans la pièce mobile 5.

Les matériaux utilisés pour la fabrication du dispositif seront naturellement choisis en fonction des différentes contraintes d'ordre mécanique et chimique auxquelles ces éléments doivent satisfaire ; ces matériaux peuvent être notamment des matières plastiques à haute résistance mécanique, des aciers inoxydables ou des alliages d'aluminium.

**Revendications**

1. Dispositif de liposuccion, du type comprenant une canule tubulaire (3), de forme cylindrique, dont une extrémité présente au moins une ouverture (30), cette canule étant raccordée à une pompe (7) qui est adaptée pour aspirer la graisse à travers ladite ouverture (30) lorsque la canule est introduite sous la peau, dans le tissu graisseux, caractérisé en ce qu'il comporte des moyens (9, 8) d'entraînement en rotation de ladite canule (3) autour de son propre axe (X-X').

2. Dispositif selon la revendication 1, caractérisé en ce que ladite canule (3) est montée, de manière amovible, à une extrémité d'une pièce tournante (5) de forme générale tubulaire qui est montée et guidée en rotation dans un support creux (2).

3. Dispositif selon la revendication 2, caractérisé en ce que la canule (3) est disposée dans le prolongement de la pièce tournante tubulaire (5), de sorte que leurs espaces intérieurs (34, 54) communiquent directement l'un avec l'autre.

4. Dispositif selon la revendication 3, caractérisé en ce que le support creux (2) présente une cavité interne (20) destinée à être raccordée à la pompe (7), qui constitue une chambre d'aspiration, et que la paroi de ladite pièce tournante tubulaire (5) présente des ouvertures (50) qui font communiquer son espace intérieur (54) avec cette chambre d'aspiration (20).

5. Dispositif selon la revendication 4, caractérisé en ce que le support creux (2) est muni d'une tubulure (24) permettant le branchement d'un tuyau (6) de raccordement de la chambre d'aspiration (20) avec la pompe (7).

6. Dispositif selon l'une des revendications 4 ou 5, caractérisé en ce que le support creux (2) a une forme extérieure approximativement cylindrique qui permet une prise en main commode du dispositif, ce support jouant le rôle de manche pour la canule (3).

7. Dispositif selon l'une des revendications 2 à 6, caractérisé en ce que l'extrémité (52) de la pièce tournante (5) qui est opposée à l'extrémité recevant la canule (3) est adaptée pour être fixée à un arbre d'entraînement (8), par exemple à un arbre flexible, lui-même entraîné par un moteur (9).

8. Dispositif selon la revendication 7, caractérisé en ce que le moteur d'entraînement (9) est un moteur possèdant deux sens possibles de rotation, dont la commande est réalisée au moyen de pédales (91, 92, 93) par l'intermédiaire d'un variateur de vitesse (90).

9. Dispositif selon l'une des revendications 2 à 8, caractérisé en ce qu'il est équipé d'un jeu de canules de longueurs et de diamètres différents, qui possèdent un embout identique (31) permettant de les monter sélectivement à l'extrémité de la pièce tournante (5).

FIG_1

FIG_2

FIG_2A

EP 0 315 557 A1

# FIG_3

# FIG_4

EP 0 315 557 A1

Office européen
des brevets

# RAPPORT DE RECHERCHE EUROPEENNE

Numero de la demande

EP 88 46 0025

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | EP-A-0 189 807 (DYONICS INC) <br> * page 6, ligne 16 - page 7, ligne 18; revendications 1, 2, 10; figures 1, 6 * <br> --- | 1, 2, 8, 9 | A 61 M 1/00 |
| Y | US-A-4 536 180 (JOHNSON) <br> * revendication 1; figures 1 - 5 * <br> --- | 1, 2, 8, 9 | |
| A | EP-A-0 190 000 (PRECISION SURGICAL INSTRUMENTS INC) <br> * revendications 1, 2, 4; figures 4, 10, 11 * <br> ----- | 1, 4 - 6 | |

### DOMAINES TECHNIQUES RECHERCHES (Int. Cl.4)

A 61 M 1/00
A 61 C 17/00
A 61 B 17/00

Le présent rapport a été établi pour toutes les revendications

| Lieu de la recherche | Date d'achèvement de la recherche | Examinateur |
|---|---|---|
| BERLIN | 07-02-1989 | MONNE E.M.B. |

EPO FORM 1503 03.82 (P0402)